(19) **Europäisches Patentamt** / **European Patent Office** / **Office européen des brevets**

(11) **EP 4 782 038 A1**

(12) # DEMANDE DE BREVET EUROPEEN

(43) Date de publication:
**29.07.2026 Bulletin 2026/31**

(21) Numéro de dépôt: **26150403.9**

(22) Date de dépôt: **06.01.2026**

(51) Classification Internationale des Brevets (IPC):
**A61M 16/00** (2006.01)    **A61M 16/20** (2006.01)

(52) Classification Coopérative des Brevets (CPC):
**A61M 16/0069; A61M 16/024; A61M 16/201; A61M 16/208;** A61M 16/0858; A61M 16/205; A61M 16/206; A61M 2016/0015; A61M 2016/0027; A61M 2016/0039; A61M 2205/502; A61M 2205/82

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **23.01.2025 FR 2500694**

(71) Demandeur: **EOVE**
**64000 Pau (FR)**

(72) Inventeurs:
• **JOURDAIN, Cedric**
  **64000 Pau (FR)**
• **LIU, Tingting**
  **64000 Pau (FR)**
• **GALBRUN, Marie-Amedee**
  **64000 Pau (FR)**
• **TEILLET, Manuel**
  **64000 Pau (FR)**

(74) Mandataire: **Air Liquide**
**L'Air Liquide S.A.**
**Direction de la Propriété Intellectuelle**
**75, Quai d'Orsay**
**75321 Paris Cedex 07 (FR)**

(54) **APPAREIL DE THÉRAPIE RESPIRATOIRE DÉLIVRANT UNE ASSISTANCE RESPIRATOIRE INTERMITTENTE PAR PRESSION POSITIVE ET DÉBIT D'INSUFFLATION DÉCROISSANT**

(57) L'invention concerne un appareil de thérapie respiratoire délivrant une assistance respiratoire intermittente par pression positive (IPPB) à une personne, comprenant des moyens de pilotage (4) sont configurés pour, lors de chaque phase inspiratoire de la personne, commander la turbine de l'appareil pour fournir un débit gazeux régulé décroissant, pendant une durée d'insufflation de gaz (Di) non-nulle suffisante pour atteindre un seuil de pression maximale ($P_{max}$) fixé.

Fig. 3

EP 4 782 038 A1

**Description**

L'invention concerne un appareil de thérapie respiratoire délivrant une assistance respiratoire intermittente par pression positive ou IPPB (pour *Intermittent Positive Pressure Breathing* en anglais), aussi appelé « appareil IPPB », avec mise en œuvre d'un débit gazeux réglé décroissant, pendant une durée d'insufflation de gaz non-nulle suffisante pour atteindre un seuil de pression maximale fixé.

D'une façon générale, fournir une thérapie respiratoire de type IPPB (appelée « thérapie IPPB » ci-après) à certaines personnes, aussi appelés « patients », souffrant de problèmes ou pathologies respiratoires permet notamment d'améliorer :

- le drainage bronchique en délivrant un volume inspiratoire important permettant d'augmenter l'efficacité du débit expiratoire et de la toux ;
- la fonction respiratoire en allant au-delà de l'inspiration maximale du patient et en augmentant ainsi sa capacité vitale ; et
- le recrutement pulmonaire, en particulier des zones pulmonaires mal ou non ventilées.

Par exemple, EP4079356 enseigne un appareil d'assistance à la toux permettant d'opérer des insufflations de gaz aux patients souffrant de troubles respiratoires nécessitant une aide pour évacuer de leurs sécrétions pulmonaires et/ou pour améliorer leur élasticité pulmonaire et thoracique, que ces patients soient des patients adultes ou pédiatriques, lequel peut être utilisé facilement par une personne n'ayant pas ou peu de connaissances médicales, typiquement une auxiliaire de vie, une personne de l'entourage familial du patient, encore appelés « les aidants ».

D'autres appareils de thérapie respiratoire sont décrits par DE102014001218, WO2015/110098, EP1502619, US2013/047983, US2024/165351 et US2008/000478.

D'une façon générale, la thérapie IPPB repose sur l'application d'un débit gazeux constant en inspiration jusqu'à atteindre une pression maximale donnée, suivi d'une expiration avec ou sans pression expiratoire positive (PEP). La synchronisation avec les phases respiratoires du patient se fait grâce à une détection de l'effort du patient, typiquement l'appel de gaz en début d'inspiration, ou par un mécanisme ou dispositif de déclenchement manuel de l'inspiration, par exemple via un appui sur un bouton ou une touche de commande.

Il existe plusieurs types de dispositifs ou d'appareils de thérapie respiratoire permettant de mettre en œuvre une thérapie IPPB mais ceux-ci présentent tous des inconvénients plus ou moins importants.

Actuellement, les dispositifs de thérapie IPPB fonctionnent sur la base d'une régulation du débit d'inspiration, c'est-à-dire du débit de gaz (e.g. air) fourni aux voies aériennes du patient pendant son traitement. Le débit fourni par la source de gaz, telle une turbine ou analogue, du dispositif ou appareil de fourniture de gaz, est habituellement constant pendant chaque phase inspiratoire du patient, ce qui se traduit par une courbe de débit de forme rectangle. Maintenir le débit fourni constant permet un remplissage progressif des poumons du patient et une augmentation progressive simultanée de la pression gazeuse dans les voies aériennes, en particulier dans les poumons du patient.

La/chaque phase inspiratoire du patient se termine lorsqu'une pression maximale préfixée est atteinte, i.e. un seuil de pression maximale réglable. Autrement dit, pour terminer l'inspiration (i.e. une phase inspiratoire), on utilise le seuil de pression qui a été réglé par l'utilisateur, typiquement un personnel soignant, qui permet de déclencher un passage en expiration (i.e. phase expiratoire) lorsqu'il est atteint. Procéder ainsi vise à mieux contrôler la pression maximale de coupure pour maximiser le confort du patient et limiter les risques de barotraumatisme.

D'une façon générale, l'obtention d'un volume maximal dans les poumons du patient est le but de la séance de traitement et l'augmentation de la pression de coupure est un moyen d'y parvenir.

Cependant, il a été constaté en pratique que l'efficacité du traitement et/ou le confort des patients, ainsi que l'observance au traitement, dépendaient notamment des physiologies des patients eux-mêmes, notamment en termes de compliance et résistance, mais aussi du type d'interface respiratoire utilisée pour administrer le gaz, i.e. masque respiratoire avec ou sans fuites.

Un problème est dès lors de proposer un appareil ou dispositif de thérapie respiratoire permettant de délivrer une assistance respiratoire intermittente par pression positive, c'est-à-dire une thérapie de type IPPB, ou « appareil IPPB », qui soit amélioré, notamment qui permette de palier tout ou partie des inconvénients susmentionnés, en particulier de délivrer une thérapie IPPB plus efficace avec un **confort accru** pour le patient tout en améliorant le remplissage des poumons du patient avec le gaz insufflé, voire aussi son observance au traitement.

Une solution selon l'invention concerne un appareil (i.e. dispositif ou installation) de thérapie respiratoire, c'est-à-dire un « appareil IPPB », pour fournir une assistance respiratoire intermittente par pression positive (IPPB) à une personne, i.e. un patient, comprenant :

- une coque ou carcasse périphérique,
- une turbine motorisée (i.e. turbine, compresseur, (micro)soufflante ou analogue) pour fournir un gaz respiratoire sous pression,
- un circuit de gaz interne pour convoyer le gaz respiratoire délivré par la turbine,
- un capteur de débit agencé sur le circuit de gaz interne,
- un dispositif anti-retour agencé dans le circuit de gaz interne, en aval du capteur de débit,
- un circuit patient externe venant se raccorder fluidiquement au circuit de gaz interne pour convoyer le gaz respiratoire provenant du circuit de gaz interne,
- un capteur de pression proximale agencé pour permettre une mesure de la pression (i.e. des mesures de pression instantanée) au sein du circuit patient externe à proximité de la valve expiratoire, et
- des moyens de pilotage raccordés au capteur de pression proximale et à la turbine motorisée, et configurés pour commander la turbine en fonction d'au moins une mesure de pression opérée par le capteur de pression proximale et d'au moins une mesure de débit opérée par le capteur de débit.

La turbine, les moyens de pilotage, le circuit interne, le capteur de débit et le dispositif anti-retour sont agencés dans la coque ou carcasse périphérique.

De plus, dans l'appareil IPPB, les moyens de pilotage sont configurés pour, lors de chaque phase inspiratoire de la personne, i.e. du patient, commander la turbine pour fournir un débit gazeux régulé décroissant, pendant une durée d'insufflation de gaz (Di) non-nulle suffisante pour atteindre un seuil de pression maximale ($P_{max}$) fixé.

Selon le mode de réalisation considéré, l'appareil de l'invention, c'est-à-dire « l'appareil IPPB », peut comprendre l'une ou plusieurs des caractéristiques suivantes :

- le débit gazeux régulé décroit proportionnellement à l'augmentation de la pression jusqu'à atteindre le seuil de pression maximale ($P_{max}$).
- les moyens de pilotage sont configurés pour, lors de chaque phase inspiratoire de la personne, commander la turbine pour fournir un débit gazeux régulé décroissant entre un débit initial ($Q_{init}$) et un débit final ($Q_{fin}$), avec $Q_{init} > Q_{fin}$.
- le débit initial ($Q_{init}$) est égal à : y.($Q_{réglé}$) avec y compris entre 0,5 et 2, de préférence entre 1 et 2.
- le débit gazeux réglé ($Q_{réglé}$) est choisi par l'utilisateur, de préférence via une ou des touches de réglage par exemple ou analogues.
- la ou les touches de réglage sont de préférence une ou des touches virtuelles s'affichant sur un écran tactile, par exemple un écran faisant partie d'une interface homme-machine (IHM).
- le débit final ($Q_{fin}$) est égal à une proportion (x%) du débit initial ($Q_{init}$) comprise entre 0 et 70% du débit initial ($Q_{init}$), c'est-à-dire $Q_{fin} = x\% . Q_{init}$ avec x% compris entre 0% (i.e. débit nul) et 70%.
- le débit final ($Q_{fin}$) est égal à une proportion (x%) du débit initial ($Q_{init}$) comprise entre 10 et 70% du débit initial ($Q_{init}$), c'est-à-dire $Q_{fin} = x\% . Q_{init}$ avec x% compris entre 10 et 70%, de préférence encore entre 20 et 70%.
- avantageusement, le débit final ($Q_{fin}$) est égal à une proportion (x%) du débit initial ($Q_{init}$) comprise entre 30 et 70% du débit initial (Qinit), c'est-à-dire $Q_{fin} = x\% . Q_{init}$ avec x% compris entre 30 et 70%.
- selon un mode de réalisation, le débit final ($Q_{fin}$) est égal à une proportion (x%) du débit initial ($Q_{init}$) comprise entre 40 et 60% du débit initial ($Q_{init}$).
- selon un mode de réalisation, le débit final ($Q_{fin}$) est égal à une proportion (x%) du débit initial ($Q_{init}$) comprise entre entre 45 et 70% du débit initial (Qinit).
- selon un mode de réalisation, le débit final ($Q_{fin}$) est égal à une proportion (x%) du débit initial ($Q_{init}$) de l'ordre de 50% du débit initial ($Q_{init}$).
- de préférence, le débit objectif ($Q_{objectif}$) est égal à une proportion du débit initial ($Q_{init}$) et par ailleurs à la montée en pression du circuit patient selon la formule suivante :

$$Q_{objectif} = (100\% - x\%) . Q_{init} . (P_{max} - P_{mes}) / P_{max} + x\% . Q_{int}$$

où :

- $Q_{objectif}$ correspond à la valeur de débit régulée au cours de l'inspiration considérée.
- $Q_{init}$ correspond à la valeur de débit initial, c'est-à-dire un débit réglé ($Q_{réglé}$), de préférence le débit initial ($Q_{init}$) est égal à : y.($Q_{réglé}$) avec y compris entre 0,5 et 2, de préférence entre 1 et 2.
- x% correspond à la proportion du débit initial ($Q_{init}$) comprise entre 0 et 70%, de préférence entre 30 et 70%, par exemple de l'ordre de 50%,
- $P_{max}$ est la pression maximale fixée.
- $P_{mes}$ est la pression proximale mesurée par le capteur de pression proximale.

- le débit objectif correspond à la valeur de débit théorique que l'appareil va utiliser pour réguler le débit délivré par la turbine.

- les moyens de pilotage sont configurés pour commander la turbine, i.e. son moteur, pour que le débit gazeux régulé fourni soit un débit progressivement décroissant pendant la durée d'insufflation de gaz (Di), c'est-à-dire diminuant entre la valeur de débit initial ($Q_{init}$) et la valeur de débit final ($Q_{fin}$), avec $Q_{init} > Q_{fin}$.
- pendant la durée d'insufflation de gaz (Di), la turbine est pilotée en débit de sorte de fournir le débit gazeux progressivement décroissant souhaité.
- bien entendu, le débit réellement fourni par la turbine peut présenter de légères variations ou fluctuations autour de la (ou des) valeur(s) de débit souhaitée, par exemple des fluctuations de l'ordre +/-5% ou moins.
- les moyens de pilotage sont configurés pour commander le moteur électrique de la turbine pour fournir le ou les débit gazeux et/ou pression désirés.
- le moteur électrique de la turbine est préférentiellement sans balai et/ou à courant continu.
- selon un mode de réalisation particulier, lorsqu'une pression mesurée ($P_{mes}$) par le capteur de pression proximale est ou devient supérieure ou égale au seuil de pression maximale ($P_{max}$) préfixé (i.e. $P_{mes} = P_{max}$), les moyens de pilotage sont configurés pour commander la turbine pour fournir (ou maintenir) une pression de plateau ($P_{plat}$) correspondant au seuil de pression maximale ($P_{max}$) de manière à maintenir au moins une partie du circuit patient externe à la pression de plateau ($P_{plat}$), pendant une durée de plateau (Dp) non-nulle donnée.
- des moyens de réglage de durée permettent à l'utilisateur de régler la durée de plateau (Dp), de préférence entre 1 et 10 sec, typiquement entre 0,5 et 5 sec.
- pendant la durée d'insufflation de gaz (Di), la turbine (i.e. son moteur) est pilotée en débit, alors qu'ensuite, pendant la durée de plateau (Dp), elle est pilotée en pression.
- il comprend des moyens de réglage de pression configurés pour permettre à l'utilisateur de régler le seuil de pression maximale ($P_{max}$).
- de préférence, le seuil de pression maximale ($P_{max}$) est compris entre 10 et 50 cmH2O.
- il comprend des moyens de réglage de débit pour permettre à l'utilisateur de régler la valeur de débit ($Q_{réglé}$) désirée, typiquement la valeur de débit initial ($Q_{init}$).
- de préférence, la valeur de débit ($Q_{réglé}$), typiquement la valeur de débit initial ($Q_{init}$), est comprise entre 1 et 200 L/min, de préférence entre 3 et 150 L/min, préférentiellement encore entre 5 et 100 L/min.
- les moyens de réglage de durée et/ou les moyens de réglage de pression et/ou de réglage de débit comprennent un ou des boutons, touches ou analogues, en particulier un ou des boutons affichés sur un écran tactile.
- les moyens de pilotage sont configurés pour comparer une valeur de pression proximale mesurée ($P_{mes}$) par le capteur de pression proximale au seuil de pression maximale ($P_{max}$) fixé afin de déterminer la fin de la durée d'insufflation de gaz (Di), c'est-à-dire la fin de l'insufflation de gaz à débit décroissant.
- une valve expiratoire est agencée sur le circuit patient externe.
- à la fin de la durée d'insufflation de gaz (Di), ou, si elle est présente, à la fin de la durée de plateau (Dp) suivant la période d'insufflation de gaz, la valve expiratoire s'ouvre pour évacuer vers l'atmosphère au moins une partie de la pression gazeuse présente dans le circuit patient externe.
- la valve expiratoire s'ouvre en réponse à une baisse de pression dans le circuit de gaz interne.
- une ligne de pilotage pneumatique de la valve expiratoire vient se raccorder fluidiquement au circuit de gaz interne entre la turbine et le capteur de débit.
- la ligne de pilotage pneumatique coopère avec la valve expiratoire pour contrôler l'ouverture ou la fermeture de ladite valve expiratoire.
- l'ouverture de la valve expiratoire permet une évacuation de gaz, i.e. de pression gazeuse, du circuit patient externe vers l'atmosphère ambiante et une diminution de pression dans tout ou partie du circuit patient externe.
- une ligne de pilotage pneumatique est une liaison pneumatique, typiquement un tuyau flexible, de préférence en polymère.
- les moyens de pilotage sont raccordés au capteur de pression proximale, c'est-à-dire que le capteur de pression peut être, selon le mode de réalisation considéré, relié électriquement auxdits moyens de pilotage et/ou être (directement) intégrés auxdits moyens de pilotage, de manière à coopérer avec lesdits moyens de pilotage.
- de préférence, le capteur de pression proximale est intégré aux moyens de pilotage, en particulier porté par une carte électronique faisant partie des moyens de pilotage.
- le capteur de pression proximale comprend une ligne de mesure de pression venant se raccorder au circuit patient en aval de la valve expiratoire.
- le capteur de pression proximale est configuré pour permettre une mesure de la pression au sein du circuit patient externe entre la valve expiratoire et une interface respiratoire assurant les échanges gazeux avec le patient, typiquement lors des phases inspiratoires, et/ou éventuellement lors des phases expiratoires dudit patient.
- le circuit de gaz interne comprend un passage, un conduit ou analogue servant à véhiculer du gaz.
- l'interface respiratoire est un masque respiratoire, une sonde de trachéostomie ou un embout buccal.
- le circuit de gaz interne comprend une sortie de gaz.

- le circuit patient externe vient se raccorder fluidiquement à ladite sortie de gaz du circuit de gaz interne.
- le circuit patient externe comprend un tuyau flexible, typiquement en polymère.
- le circuit patient externe peut aussi comprendre ou mettre en oeuvre des éléments de connexion fluidique additionnels de type connecteurs, raccords ou analogues.
- le circuit patient externe comprend une branche unique ou simple branche
- selon un autre mode de réalisation, le circuit patient externe peut être à double branche.
- les moyens de pilotage comprennent au moins un microprocesseur.
- ledit au moins un microprocesseur est agencé sur au moins une carte électronique.
- les moyens de pilotage comprennent au moins une carte électronique.
- la turbine motorisée comprend un moteur électrique.
- le moteur électrique est alimenté électriquement (110/220V).
- les moyens de pilotage sont configurés pour commander les accélérations du moteur électrique de la turbine, et/ou les décélérations ou freinages dudit moteur. Par décélération, on entend un freinage ou ralentissement du moteur, par exemple opéré par inversion des phases du moteur.
- le dispositif anti-retour comprend un clapet anti-retour, une valve anti-retour ou analogue.
- le dispositif anti-retour est configuré pour s'opposer à tout flux négatif, c'est-à-dire remontant en direction de la turbine, i.e. s'écoulant dans le sens opposé au sens normal de circulation du gaz de la turbine vers le patient.
- la valve expiratoire est préférentiellement une valve à 3 voies.
- selon un autre mode de réalisation, la valve expiratoire peut être une valve de PEP.
- le moteur électrique de la turbine est piloté, i.e. commandé ou contrôlé, par les moyens de pilotage.
- la ligne de pilotage pneumatique comprend un tuyau flexible en polymère de petit diamètre, typiquement d'environ 2 à 10 mm de diamètre.
- la coque ou carcasse périphérique est formée d'une ou plusieurs sous-parties fixées entre elles, par exemple par vissage.
- la coque ou carcasse périphérique peut être en polymère rigide.
- il comprend une interface graphique utilisateur ou IGU, aussi appelée interface homme-machine ou IHM.
- l'IGU peut comprendre un écran d'affichage, de préférence de type tactile, et/ou des touches de sélection ou de validation, notamment des touches tactiles affichées sur l'écran.
- il comprend des moyens de mémorisation, telle une mémoire RAM ou EEPROM, ou une mémoire flash, ou autre.
- les moyens de pilotage commandent un ralentissement ou un freinage de la turbine lorsque lesdits moyens de pilotage déterminent que la pression proximale mesurée, typiquement par le capteur de pression proximal situé au plus près du patient, atteint une valeur de pression maximale (pré)réglée, à savoir le seuil de pression maximale ($P_{max}$).
- les moyens de pilotage sont configurés pour comparer la valeur de pression proximale mesurée par le capteur de pression proximale à la valeur de pression maximale (pré)réglée, c'est-à-dire le seuil de pression maximale ($P_{max}$), et en déduire la fin de la durée d'insufflation de gaz (Di), la fin d'un temps d'insufflation($T_{max}$) maximal, et/ou le début d'une durée de plateau (Dp) éventuelle.
- lorsqu'une durée de plateau (Dp) débute après la phase d'insufflation, cette durée de plateau est (pré)fixée, i.e. réglée par l'utilisateur (i.e. personnel médical), de préférence via l'IGU, et/ou mémorisée par les moyens de mémorisation.
- le moteur de la turbine est configuré pour pouvoir tourner à une vitesse de rotation pouvant atteindre 50 000 tours/min, voire 70 000 tr/min.
- les moyens de pilotage comprennent un compteur temporel permettant de déterminer ou mesurer les durées.
- le seuil de pression maximale ($P_{max}$) est (pré)fixé, i.e. réglé par l'utilisateur (i.e. personnel médical), de préférence via l'IGU ou IHM.
- le seuil de pression maximale ($P_{max}$) est mémorisé par les moyens de mémorisation.
- la valve d'expiration, i.e. valve expiratoire, est configurée pour s'ouvrir automatiquement dès lors qu'une dépression se crée dans le circuit interne, i.e. en amont du clapet qui alimente la valve, du fait d'une décélération (i.e. freinage) de la turbine, i.e. c'est-à-dire lorsque (le moteur de) la turbine ralentit, en particulier après la phase d'insufflation, ou après une phase de plateau de pression éventuelle. Ceci est possible grâce au raccordement fluidique de la ligne de pilotage de la valve d'expiration en aval de la turbine et en amont du dispositif anti-retour, c'est-à-dire entre turbine et dispositif anti-retour de gaz, et au pilotage pneumatique (i.e. en pression) de ladite valve d'expiration par ladite ligne de pilotage.
- la valve d'expiration comprend un corps de valve comprenant des moyens d'étanchéité flexibles.
- les moyens d'étanchéité flexibles comprennent une membrane, un ballonnet ou analogue, de préférence flexible et/ou déformable, typiquement une membrane.
- la ligne de pilotage pneumatique contrôle l'ouverture et/ou la fermeture de la valve d'expiration en agissant pneumatiquement, c'est-à-dire via une fourniture de gaz sous pression, sur les moyens d'étanchéité flexibles du corps de valve.

- le corps de valve de la valve d'expiration comprend au moins un orifice de mise à l'atmosphère.
- les moyens d'étanchéité flexibles sont configurés pour autoriser ou, à l'inverse, pour empêcher une circulation du flux gazeux au travers de l'orifice de mise à l'atmosphère, c'est-à-dire une décharge de gaz à l'atmosphère.
- les moyens d'étanchéité flexibles sont agencés sur un passage de flux gazeux traversant le corps de valve.
- les moyens d'étanchéité flexibles sont configurés pour coopérer avec au moins une partie de la paroi du corps de valve pour contrôler ou assurer l'étanchéité gazeuse, en particulier au moins une partie de la paroi du passage de flux gazeux agencé dans le corps de valve.
- le circuit de gaz interne comprend une sortie de gaz, par exemple portée par un raccord ou embout de sortie ou analogue.
- le circuit patient externe vient se raccorder fluidiquement à la sortie de gaz du circuit de gaz interne, par exemple vient se connecter au raccord ou embout de sortie ou analogue du circuit de gaz interne.
- selon un mode de réalisation, lorsqu'une durée de plateau suit la phase d'insufflation, les moyens de pilotage contrôlent la turbine à la fin de la phase d'insufflation de sorte que, durant la durée de plateau Dp, le débit délivré par la turbine diminue progressivement. Un débit gazeux résiduel est maintenu pendant au moins une partie de la durée de plateau Dp avec maintien, par ailleurs de la pression à $P_{max}$. Pendant la durée de plateau Dp, la valve expiratoire est fermée de manière à maintenir d'au moins une partie du circuit patient externe à la pression de plateau $P_{pla}$, et à la fin de la durée de plateau Dp, la valve expiratoire s'ouvre de manière à évacuer au moins une partie de la pression gazeuse présente dans le circuit patient externe.
- la valve expiratoire est maintenue fermée pendant la durée d'inspiration Di et pendant la durée de plateau Dp optionnelle.

Par ailleurs, l'invention concerne aussi une méthode pour fournir une thérapie respiratoire de type IPPB à une personne en ayant besoin, i.e. un patient, dans laquelle on utilise un appareil (i.e. dispositif ou installation) de thérapie respiratoire, comme décrit ci-avant ou ci-après, pour fournir un gaz respiratoire à ladite personne, dans laquelle, lors de chaque phase inspiratoire de la personne, i.e. du patient, on fournit un débit gazeux régulé décroissant, pendant une durée d'insufflation de gaz (Di) non-nulle suffisante pour atteindre un seuil de pression maximale ($P_{max}$) fixé.

La fourniture du débit gazeux régulé décroissant est opérée par commande de la turbine de l'appareil, i.e. par les moyens de pilotage.

Dans le cadre de la présente invention:

- on appelle « capteur de pression », tout dispositif ou moyen permettant de déterminer une pression de gaz.
- on appelle « capteur de débit », tout dispositif ou moyen permettant de déterminer un débit de gaz, y compris indirectement via des mesures de pression.
- les termes « appareil » et « dispositif » sont considérés comme équivalents et substituables.
- les termes « turbine », « compresseur », « soufflante » ou analogue sont considérés comme équivalents et substituables.
- les termes « valve » et « vanne » sont considérés comme équivalents et substituables.
- les termes « moyens » et « dispositif » sont considérés comme équivalents et substituables, par exemple les termes « moyens de pilotage » sont considérés comme équivalents et substituables par les termes « dispositif de pilotage ».
- les termes « piloté », « commandé » et « contrôlé » sont considérés comme équivalents et substituables.
- les termes « piloter », « commander » et « contrôler » sont considérés comme équivalents et substituables
- on considère que la respiration d'une personne comprend ou est formée de cycles respiratoires successifs, chaque cycle respiratoire comprenant une phase inspiratoire suivie d'une phase expiratoire.
- pendant une phase inspiratoire, une personne inhale/inspire dans ses poumons du gaz respiratoire, tel de l'air ou un mélange air/$O_2$.
- pendant une phase expiratoire, une personne exhale/expire de ses poumons du gaz contenant du $CO_2$.

L'invention va maintenant être mieux comprise grâce à la description détaillée suivante, faite à titre illustratif mais non limitatif, en référence aux figures annexées parmi lesquelles :

Fig. 1 est un schéma de principe d'un mode de réalisation d'un appareil de thérapie respiratoire selon l'invention.
Fig. 2 schématise (vue extérieure partielle) un mode de réalisation d'un appareil selon l'invention, tel celui de Fig. 1, avec son raccordement au secteur.
**Fig. 3 schématise** des courbes de débit (Q) et pression (P) obtenues avec un appareil IPPB piloté selon l'invention (INVENTION) et, à titre comparatif, selon l'art antérieur (ART ANTERIEUR).
Fig. 4 schématise des courbes de débit (Q) et pression (P) obtenues avec un appareil IPPB piloté selon l'invention, comme illustré en Fig. 3, mais comprenant une phase de maintien d'un plateau de pression en fin de la durée d'insufflation.

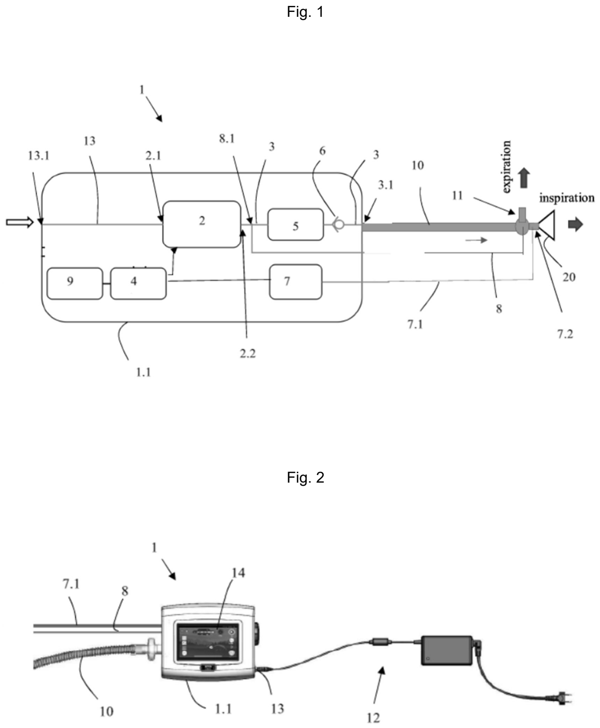

Fig. 1 et Fig. 2 schématisent un mode de réalisation d'un appareil ou dispositif 1 de thérapie respiratoire selon l'invention permettant de fournir une assistance respiratoire intermittente par pression positive, appelée « thérapie IPPB » ci-après, à une personne en ayant besoin, à savoir typiquement un patient à traiter ou analogue.

Comme illustré sur le schéma de principe de Fig. 1, l'appareil de thérapie respiratoire 1 ou « appareil IPPB » comprend une turbine 2 motorisée (aussi appelée (micro-)soufflante, pompe, compresseur ou analogue) combinée à un système de commande passif comprenant d'une valve d'expiration 11, aussi appelée valve expiratoire, et à des moyens de mesure de pression proximale, typiquement un capteur de pression proximale 7, comme décrit ci-après, dans le but de délivrer la thérapie IPPB au patient de manière efficace et avec un confort accru pour le patient, notamment en termes de limitation ou de réduction des nuisances sonores, c'est-à-dire du bruit généré lors du fonctionnement de l'appareil 1.

Plus précisément, la turbine 2 motorisée comprend un moteur électrique, typiquement sans balai, qui est piloté, i.e. contrôlé, par des moyens de pilotage 4, typiquement par un (des) microprocesseur agencé sur une (des) carte électronique. La turbine 2 est alimentée en air ambiant par une ligne d'amenée d'air 13, tel un conduit, un passage ou analogue, qui relie une entrée d'air 13.1 à l'entrée 2.1 de la turbine 2.

La turbine 2 motorisée a une architecture classique, à savoir qu'elle comprend schématiquement un moteur électrique agencé dans un carter de protection, lequel est surmonté d'une volute comprenant un compartiment interne dans lequel est agencée une roue à ailettes. La roue à ailettes est portée par l'axe du moteur. Elle est entrainée en rotation par l'axe du moteur, lors de son fonctionnement, de manière à d'aspirer le gaz respiratoire, tel de l'air, qui pénètre alors dans le compartiment interne de la volute, puis en ressort avant d'être envoyé vers le patient.

La turbine 2 fournit ensuite le gaz respiratoire sous pression, tel de l'air, à un circuit de gaz 3 interne servant à acheminer, i.e. convoyer, le gaz respiratoire délivré en sortie 2.2 de turbine 2 jusqu'à une interface respiratoire 20, tel un masque respiratoire, un embout buccal ou une sonde de trachéostomie, fournissant le gaz respiratoire aux voies aériennes du patient à traiter.

Lorsque le moteur électrique de la turbine 2 (i.e. sa vitesse de rotation) est contrôlé par les moyens de pilotage 4, son axe portant la roue à ailettes est entrainé en rotation, ce qui permet de fournir le gaz au débit souhaité, par exemple au débit réglé ($Q_{réglé}$), selon le contrôle opéré, comme expliqué ci-après.

A l'inverse, les moyens de pilotage 4 peuvent aussi contrôler la vitesse de rotation du moteur pour le freiner ou le ralentir. Dans ce cas, la vitesse de rotation de l'axe-moteur portant la roue à ailettes diminue plus ou moins rapidement et la fourniture de gaz diminue alors en corrélation avec le ralentissement/freinage du moteur.

Un exemple de turbine ou (micro-)soufflante pour appareil médical de ventilation est donné par EP2947328 mais, bien entendu, d'autres types de turbines peuvent convenir.

Utiliser une turbine motorisée 2 pour délivrer le gaz permet d'obtenir une limitation du bruit généré pendant le fonctionnement de l'appareil 1.

Afin de convoyer le gaz respiratoire provenant du circuit de gaz 3 interne, c'est-à-dire agencé dans la coque 1.1 de l'appareil 1, jusqu'à l'interface respiratoire 20, on prévoit un circuit patient 10 externe, typiquement un (des) conduit ou tuyau flexible, qui vient se raccorder fluidiquement à une sortie de gaz 3.1 du circuit de gaz 3 interne, typiquement un tuyau flexible en polymère. La sortie de gaz 3.1 peut être portée par un connecteur ou raccord de sortie agencé sur la carcasse 1.1 de l'appareil 1. Le circuit patient 10 externe vient se raccorder fluidiquement audit raccord de sortie, par exemple s'y emboiter, s'y visser, s'y fixer via un système à baïonnette ou tout autre système de raccordement adapté, de préférence le tuyau flexible comprend des moyens de connexion fluidique complémentaires du raccord de sortie.

Afin de mesurer le débit de gaz au sein de l'appareil 1, un capteur de débit 5 est agencé sur le circuit de gaz 3 interne, c'est-à-dire en aval de la sortie de la turbine 2 et en amont de la sortie de gaz 3.1 du circuit de gaz 3. Le capteur de débit 5 fournit les mesures de débit aux moyens de pilotage 4, où ils sont traités afin de réguler le fonctionnement de la turbine 2, typiquement les accélérations ou le freinage de son moteur électrique. Le capteur de débit 5 est raccordé électriquement aux moyens de pilotage 4, typiquement par des liaisons électriques, tels des câbles électriques ou analogues, afin de lui transmettre les mesures de débit réalisées.

On peut aussi utiliser ces mesures de débit pour vérifier le bon fonctionnement du dispositif anti-retour 6, e.g. clapet anti-retour, en s'assurant qu'il n'existe aucun flux négatif remontant vers la turbine 2, lors des phases expiratoires du patient, c'est-à-dire de vérifier que le dispositif anti-retour 6 garantit une étanchéité fluidique dans le sens négatif.

Le circuit patient 10 externe comprend, quant à lui, la valve expiratoire 11 servant à contrôler les rejets de gaz expirés par le patient et/ou tout ou partie de la pression gazeuse présente dans le circuit patient 10, en particulier après la phase de plateau comme expliqué ci-après, vers l'atmosphère ambiante étant donné que cette valve expiratoire 11 permet de mettre en relation la partie aval du circuit patient 10 avec l'extérieur, c'est-à-dire l'atmosphère ambiante, en particulier en fin de phase inspiratoire et/ou lors des phases expiratoires du patient.

Par ailleurs, afin d'empêcher les remontées de gaz/pression et permettre leur meilleure évacuation via la valve expiratoire 11, un dispositif anti-retour 6, c'est-à-dire un clapet anti-retour, une valve unidirectionnelle ou analogue, est agencé sur le circuit de gaz 3 interne, en aval du capteur de débit 5 et en amont de la sortie de gaz 3.1 du circuit de gaz 3. Un tel dispositif anti-retour 6 présente en outre l'avantage d'être simple à mettre en œuvre et de ne demander aucun pilotage particulier, donc d'être aussi de coût bas.

La présence de ce dispositif anti-retour 6, typiquement un clapet anti-retour, permet aussi d'améliorer le fonctionnement de l'appareil 1, typiquement l'ouverture de la valve expiratoire 11 lors des phases expiratoires, comme expliqué ci-après.

La valve expiratoire 11, en particulier son ouverture et/ou sa fermeture, est commandée ou contrôlée par une ligne de pilotage pneumatique 8, tel un conduit flexible, venant se raccorder fluidiquement 8.1 au circuit de gaz 3 interne entre la turbine 2 et le capteur de débit 5. La ligne de pilotage pneumatique 8 coopère avec la valve expiratoire 11 en lui fournissant une pression gazeuse servant à contrôler l'ouverture ou la fermeture de ladite valve expiratoire 11, c'est-à-dire son degré d'ouverture/fermeture.

Préférentiellement, l'ouverture et/ou la fermeture de ladite valve expiratoire 11 est/sont contrôlée(s) par des moyens d'étanchéité flexibles sur lesquels agit la pression gazeuse apportée par la ligne de pilotage pneumatique 8, comme expliqué ci-après.

Enfin, le circuit patient 10 externe comprend aussi un capteur de pression proximale 7 agencé de manière à permettre une mesure de la pression, i.e. de la pression instantanée, au sein du circuit patient 10 et à proximité 7.2 de la valve expiratoire 11, typiquement entre la valve expiratoire 11 et l'interface respiratoire 20.

Ce capteur de pression proximale 7 permet de détecter les appels de gaz du patient, c'est-à-dire ses inspirations, mais aussi si le seuil de pression maximale ($P_{max}$) fixé a été atteint en fin de phase inspiratoire, comme détaillé ci-après.

Le capteur de pression proximale 7 est raccordé aux moyens de pilotage 4, c'est-à-dire qu'il coopère avec ceux-ci, de sorte que ces derniers puissent piloter la turbine 2 à partir de tout ou partie des mesures opérées par le capteur de pression proximale 7. De préférence, le capteur de pression proximale 7 est directement intégré aux moyens de pilotage 4, par exemple porté par une carte électronique desdits moyens de pilotage 4.

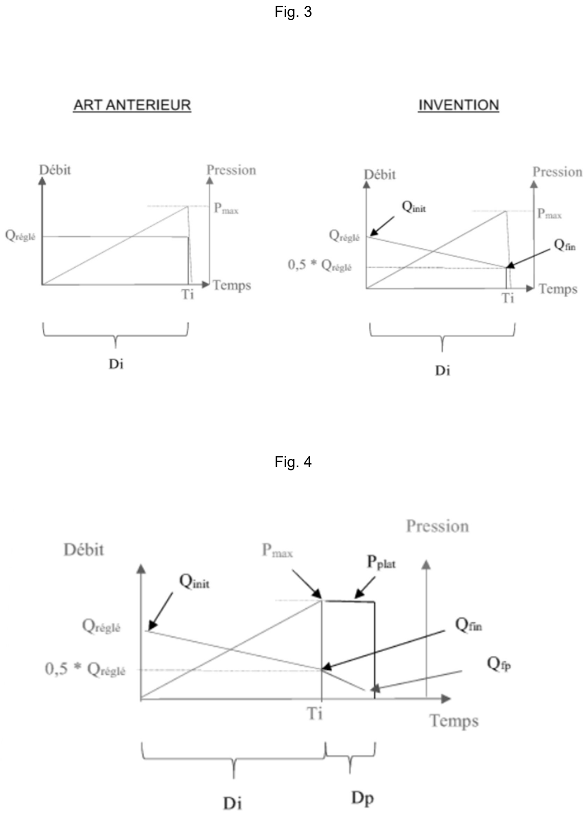

L'utilisation d'une turbine motorisée 2 pilotée assure un confort accru tant au niveau du plus faible bruit généré par l'appareil 1 lors d'un traitement IPPB, que de l'adaptabilité du profil de débit délivré au patient, par exemple un débit en « rectangle », comme illustré sur Fig. 3, ou autre.

Par ailleurs, la mesure de la pression proximale, via le capteur de pression proximale 7 apporte une performance supplémentaire au dispositif 1, grâce à la grande précision des mesures de pression opérées, et aussi un confort d'utilisation pour le patient, grâce à une meilleure détection de l'effort respiratoire du patient, qui permettent d'obtenir une synchronisation efficace, voire (quasi-)optimale, entre appel de gaz par le patient et fourniture de gaz par la turbine lors des phases inspiratoires, et à l'inverse, le rejet des gaz expirés par la valve expiratoire 11.

Comme illustré en Fig. 2, l'appareil 1 comprend aussi des moyens de réglage ou de sélection, telles des touches virtuelles affichées par l'afficheur, i.e. écran digital 14, d'une interface graphique utilisateur 9 ou IGU. L'écran d'affichage 14 est de préférence de type tactile et/ou à affichage en couleurs. Il comprend des touches de sélection ou de validation, notamment des touches tactiles affichées sur l'écran.

La turbine 2, les moyens de pilotage 4, le circuit interne 3, le capteur de débit 5 et le dispositif anti-retour 6 sont agencés dans la coque ou carcasse 1.1 périphérique de l'appareil 1, par exemple une carcasse en polymère rigide ou un autre matériau adapté.

D'une façon générale, sont prévus aussi des moyens d'alimentation électrique, telle une liaison électrique secteur (110/220V), permettent d'alimenter électriquement l'appareil 1, en particulier tous les composants nécessitant de l'énergie électrique pour fonctionner, tels les moyens de pilotage 4, l'IGU 9, l'écran 14, les capteurs 5, 7... De préférence, comme illustré en Fig. 2, les moyens d'alimentation électrique peuvent comprendre un ensemble 12 comprenant un (des) câble électrique avec transformateur de courant et prise de liaison au secteur (110/220V) venant se raccorder à un connecteur électrique 13 de l'appareil 1.

En outre, l'appareil 1 peut comprendre aussi des moyens de mise en route et/ou d'arrêt, comme une touche d'allumage/d'extinction (i.e. touche « On/Off » en anglais) ou équivalent.

Durant le fonctionnement de l'appareil 1, lors des phases inspiratoires ou insufflatoires du patient, la turbine 2 est pilotée pour délivrer un débit régulé décroissant selon le (les) réglage(s) ou paramètre(s) de contrôle entré(s), fixé(s) ou sélectionné(s) par l'utilisateur via l'IGU 9, en particulier une valeur de pression maximale, aussi appelée seuil de pression maximale ($P_{max}$) comme illustré en Fig. 3 et Fig. 4, et éventuellement un temps maximum d'inspiration ($T_{max}$), c'est-à-dire une durée maximale pendant laquelle du gaz est délivré au patient pendant chaque phase inspiratoire de durée Di. Selon d'autres modes de réalisation, on peut aussi sélectionner d'autres paramètres, tel que pente inspiratoire et/ou expiratoire, sensibilité de déclenchement, temps de thérapie, etc...

Plus précisément, les moyens de pilotage 4, typiquement un (des) microprocesseur, sont configurés pour commander la turbine 2, typiquement le moteur électrique de la turbine, lors de chaque phase inspiratoire du patient, pour d'abord fournir un débit gazeux ($Q_{réglé}$) contrôlé/désiré, typiquement un débit d'air, pendant une durée d'insufflation de gaz (Di) non-nulle suffisante pour atteindre un seuil de pression maximale ($P_{max}$) préfixé, par exemple compris entre 10 et 50 cmH2O.

Ainsi, Fig. 3 permet de comparer les courbes de débit (Q)/pression (P) en fonction du temps obtenues avec l'appareil IPPB 1 contrôlé en débit selon l'art antérieur (« ART ANTERIEUR ») ou selon l'invention (« INVENTION »).

Dans les deux cas, pendant l'inspiration de chaque cycle respiratoire du patient, la turbine 2 de l'appareil 1 délivre le débit gazeux désiré $Q_{réglé}$ qui est constant selon l'Art Antérieur (graphe de gauche) ou décélérant/décroissant selon l'Invention (graphe de droite), en fonction des réglages opérés via l'interface ou IGU.

Le débit régulé est délivré au patient à travers du capteur de débit 5 et du clapet anti-retour 6 de l'appareil de Fig. 1.

Le débit est régulé en boucle fermé par les moyens de pilotage 4, tel un calculateur, à partir de mesures de débit opérées en sortie/en aval de la turbine 2. La vitesse de la turbine 2 peut ainsi être adaptée par les moyens de pilotage 4 en fonction du profil de débit désiré. La régulation, par exemple de type proportionnelle intégrale dérivée (PID), permet de maintenir une différence minimale entre le profil de débit souhaité et le profil de débit résultant de l'adaptation de la vitesse turbine et ce quelle que soit la pression résultante, à condition qu'elle demeure en dessous du seuil de pression maximale $P_{max}$ de coupure de l'inspiration.

Lorsque le débit est délivré au patient, la pression P dans le circuit patient externe 10 et donc dans les poumons du patient augmente progressivement, par exemple comme ici de manière linéaire, mais une montée non-linéaire est aussi possible, jusqu'à atteindre la pression maximale $P_{max}$ préfixée et mémorisée.

Durant toute la phase inspiratoire du patient, la valve expiratoire 11 est fermée, c'est-à-dire pendant la durée Di.

Lorsque la pression instantanée mesurée ($P_{mes}$) par le capteur de pression proximale 7 atteint le seuil de pression maximale ($P_{max}$) préfixé, i.e. $P_{mes} = P_{max}$, les moyens de pilotage 4 cessent de piloter la turbine 2 en débit.

A ce moment, la turbine 2 n'est plus pilotée en débit et elle ralentit, ce qui engendre une chute du débit de gaz fourni et aussi, dans les conditions de Fig. 3, une chute rapide de la pression dans le circuit patient externe 10 et donc dans les poumons du patient puisqu'à la fin de la phase d'insufflation Di et/ou du temps maximal d'insufflation Ti, la valve d'expiration 11 s'est ouverte, ce qui engendre une évacuation du gaz vers l'atmosphère.

Dans les deux cas, la durée d'insufflation de gaz Di correspond au temps nécessaire pour augmenter progressivement la pression gazeuse dans le circuit patient 10 externe, donc aussi dans les poumons du patient, jusqu'à atteindre une pression maximale souhaitée, à savoir le seuil de pression maximale $P_{max}$ préfixé. Ici, l'augmentation de la pression gazeuse est linéaire ; toutefois, comme déjà dit, elle pourrait être non-linéaire étant donné que l'augmentation de la pression est la résultante du débit gazeux fourni aux poumons du patient, c'est-à-dire du volume de gaz qui y pénètre, lequel peut être (très) variable en fonction notamment de la compliance et/ou de la résistance des poumons du patient.

Sur le graphe de gauche (Art Antérieur) illustrant un contrôle de débit selon l'art antérieur, le débit réglé $Q_{réglé}$ par l'utilisateur et délivré par la turbine 2 au patient est maintenu constant pendant la durée d'insufflation de gaz Di, par exemple, un débit réglé $Q_{réglé}$ compris entre 5 et 100 L/min, ce qui se traduit par une courbe de forme « rectangle ».

Par contre, sur le graphe de droite (Invention) illustrant un contrôle de débit selon l'invention, le débit réglé $Q_{réglé}$ par l'utilisateur et délivré par la turbine 2 au patient n'est pas maintenu constant, pendant la durée d'insufflation de gaz Di, mais est contrôlé pour décroitre, c'est-à-dire diminuer progressivement entre une valeur initiale de débit initial $Q_{init}$ et une valeur finale de débit final $Q_{fin}$.

Là encore, le débit réglé $Q_{réglé}$ correspondant au débit initial $Q_{init}$ peut être compris entre 5 et 100 L/min.

Le débit gazeux régulé décroit alors proportionnellement à l'augmentation de la pression jusqu'à atteindre le seuil de pression maximale ($P_{max}$).

Ici, la décroissance de débit est linéaire mais elle pourrait avoir une autre forme, par exemple une forme courbe, tel qu'un polynôme du second degré ou plus, ou autre.

Afin d'opérer un contrôle de débit décroissant selon l'invention (i.e. la pente décroissante sur graphe de droite), le débit initial ($Q_{init}$) correspond au débit réglé au début de l'inspiration, par exemple 40 L/min, et va être progressivement réduit jusqu'à atteindre le débit final ($Q_{fin}$) désiré qui est inférieur au débit initial (c'est-à-dire : Qfin < Qinit).

Préférentiellement, le débit final ($Q_{fin}$) correspond ou est égal à une proportion (x%) du débit initial ($Q_{init}$), c'est-à-dire $Q_{fin} = x\%$ . $Q_{init}$, par exemple une proportion (x%) comprise entre 0 et 70% du débit initial (Qinit), avantageusement entre 30 et 70% du débit initial ($Q_{init}$). A titre d'exemple, le débit final ($Q_{fin}$) est fixé ici à environ 50% (x%=50%) du débit réglé au début de la phase d'insufflation, c'est-à-dire à 50% du débit initial ($Q_{init}$).

Selon un mode de réalisation selon l'invention, la réduction du débit est aussi préférentiellement proportionnelle à la montée en pression du circuit et des poumons, en considérant la proportion par rapport à la pression maximale selon la formule suivante :

$$Q_{objectif} = (100 - x\%) . Q_{init} . (P_{max} - P_{mes}) / P_{max} + x\% . Q_{init}$$

où :

- $Q_{objectif}$ correspond à la valeur de débit régulée au cours de l'inspiration considérée.
- $Q_{init}$ correspond à la valeur de débit initial, c'est-à-dire un débit réglé ($Q_{réglé}$), de préférence le débit initial ($Q_{init}$) est égal à : y.($Q_{réglé}$) avec y compris entre 0,5 et 2, de préférence entre 1 et 2.
- x% correspond à une proportion (par exemple x%= 50%) par rapport au débit initial

- $P_{max}$ est la pression maximale fixée
- $P_{mes}$ est la pression proximale mesurée par le capteur de pression

Dans ce cas :

- Si $P_{mes}$ = 0 alors $Q_{objectif}$ = $Q_{init}$ au début de l'inspiration, c'est-à-dire au débit initial ($Q_{init}$)
- Si $P_{mes}$ = $P_{max}$ alors $Q_{objectif}$ = x% . $Q_{init}$ en fin de l'inspiration.

Selon l'invention (graphe de droite en Fig. 3), le fait de contrôler la turbine 2 pour qu'elle délivre un débit décroissant/décélérant pendant la phase d'insufflation (pente décroissante) permet d'améliorer le confort du patient car ce profil est plus proche de sa physiologie et par ailleurs, de permettre de délivrer un volume plus important pour une même pression maximale. Le fait d'avoir un débit plus faible à la fin de l'inspiration implique que la pression résultante de la résistance du patient et des éventuels accessoires d'interface est plus faible. Donc, pour une même pression atteinte une moindre part est issue de la résistance du patient et cela permet de mieux remplir les poumons. Ceci peut aussi engendrer une meilleure observance de son traitement.

Au final, cela conduit à une amélioration du traitement global par rapport à un contrôle selon l'Art Antérieur (graphe de gauche en Fig. 3), c'est-à-dire avec délivrance d'un débit constant avec courbe « rectangle ».

Par ailleurs, Fig. 4 schématise des courbes de débit (Q) et pression (P) obtenues avec un appareil IPPB piloté selon l'invention, comme illustré en Fig. 3, mais comprenant en outre une phase de maintien d'un plateau de pression ($P_{plat}$) en fin de durée d'insufflation Di.

La phase d'insufflation de gaz (Di) est identique à celle illustrée sur Fig. 3 et ne sera pas détaillée car il suffit de se reporter aux explications ci-avant pour en connaître le détail.

Dans ce mode de réalisation, la différence majeure se situe à la fin de la phase d'insufflation, i.e. après la durée d'insufflation Di. En effet, comme visible sur Fig. 4, après la durée d'insufflation Di à débit décroissant, i.e. à la fin du temps d'insufflation ($T_{max}$) maximal, dans ce mode de réalisation, la pression est maintenue (approximativement) constante pendant une durée de plateau (Dp) non-nulle afin de maintenir au moins une partie du circuit patient 10 externe à une pression de plateau (Ppia) qui est égale au seuil de pression maximale ($P_{max}$) préfixé, i.e. $P_{pla}$ = $P_{max}$

Par exemple, la durée de plateau (Dp) peut êre comprise entre 0,1 et 10 sec, typiquement entre 0,5 et 5 sec.

Pour ce faire, les moyens de pilotage 4 commandent la turbine 2 pour fournir une pression de plateau ($P_{plat}$) correspondant au seuil de pression maximale ($P_{max}$) lorsque ce seuil a été atteint.

Autrement dit, selon l'invention, pendant chaque phase inspiratoire du patient, les moyens de pilotage 4 commandent d'abord la turbine 2 en débit pendant la durée d'insufflation de gaz (Di) puis, lorsque la pression de plateau ($P_{max}$) est atteinte, c'est-à-dire à l'issue de la durée d'insufflation de gaz Di, ils la commandent en pression pendant la durée de plateau (Dp).

Durant toute la phase inspiratoire du patient, la valve expiratoire 11 reste fermée, c'est-à-dire pendant les durées Di et Dp.

Ainsi, une fois que le seuil de pression maximale $P_{max}$ est atteint, la pression de gaz dans le circuit patient 10 peut être maintenue à la valeur du seuil de pression maximale $P_{max}$ de manière à obtenir un plateau de pression ($P_{plat}$) régnant dans le circuit patient 10, en particulier dans sa partie aval à proximité du patient et de ses voies aériennes.

Cette pression de plateau $P_{pla}$ est maintenue (approximativement) constante pendant la durée de plateau Dp, alors que, dans le même intervalle de temps, le débit continue à diminuer progressivement de la valeur finale $Q_{fin}$ reflètant le débit en fin de durée d'insufflation de gaz Di, à une valeur débit inférieure, à savoir une valeur de débit de fin de plateau $Q_{fp}$, par exemple une valeur $Q_{fp}$ nulle ou proche de la valeur nulle (i.e. 0 L/min). Cette diminution progressive, pendant la durée de plateau Dp, du débit correspond au ralentissement ou freinage de la turbine 2 qui est alors pilotée en pression (et non plus en débit) pour maintenir la pression de plateau $P_{pla}$.

Grâce à l'instauration de ce plateau de pression (Plateau), on peut (encore plus) augmenter le confort pour le patient et l'efficacité de son traitement global, étant donné que la phase de plateau de pression permet d'augmenter le volume de gaz, i.e. d'air, délivré au patient et d'améliorer ainsi la propagation du gaz dans ses poumons et ce, sans pour autant augmenter la pression maximale de coupure, c'est-à-dire la pression maximale délivrée (i.e. $P_{max}$), donc en limitant les éventuels effets secondaires du traitement de type barotrauma.

Grâce à la phase de plateau de pression, un personnel soignant, i.e. un clinicien ou analogue, peut adapter la thérapie du patient pour obtenir le meilleur compromis possible entre la pression maximale et l'efficacité du traitement.

Selon l'invention, les moyens de pilotage 4 de l'appareil 1 opèrent donc une comparaison entre les mesures de pression proximale $P_{mes}$ (i.e. pression instantanée) provenant du capteur de pression proximale 7 et le seuil de pression maximale $P_{max}$ préfixé, et déclenchent éventuellement (en fonction du mode de réalisation désiré) une phase de plateau avec maintien à la pression de plateau $P_{pla}$.

Par ailleurs, étant donné qu'à la fin de la durée de plateau Dp, on stoppe le pilotage en pression de la turbine 2, on assiste à une chute brutale de la pression régnant dans le circuit interne 3 de l'appareil 1, en particulier au niveau du piquage 8.1 de la ligne de pilotage de la valve expiratoire 11, donc au sein de la ligne de pilotage de la valve expiratoire 11 elle-même. Cette baisse brutale de pression va alors engendrer une ouverture de la valve expiratoire 11, qui était fermée jusque-là, afin d'évacuer au moins une partie de la pression gazeuse présente dans le circuit patient 10 vers l'atmosphère extérieure et d'obtenir ainsi une baisse de pression dans le circuit patient 10 et dans les poumons du patient, de manière à lui permettre d'expirer le gaz expiré riche en $CO_2$.

Le débit gazeux résiduel maintenu pendant au moins une partie de la durée de plateau Dp peut décroitre progressivement selon un profil linéaire ou non-linéaire, par exemple sensiblement parabolique ou autre.

D'une façon générale, les paramètre(s) de contrôle de l'appareil 1, en particulier le seuil de pression maximale ($P_{max}$), la durée de plateau (Dp) éventuelle et/ou le (les) débit réglé $Q_{réglé}$, en particulier $Q_{init}$, peuvent être mémorisés au sein de moyens de mémorisation de l'appareil 1, telle une mémoire flash, RAM, EEPROM ou analogue. Ces paramètres peuvent être réglés via des moyens de réglages de pression, de débit et/ou de durée, telles une ou des touches ou boutons de réglage, par exemple des touches virtuelles ou analogues affichées par l'IGU.

Dans tous les cas, comme illustré en Fig. 1, le gaz provenant de la turbine 2, tel de l'air, pendant son fonctionnement, est délivré au patient après avoir été convoyé par le circuit de gaz 3 interne, donc via le capteur de débit 5 et le dispositif anti-retour 6 (i.e. clapet anti-retour ou analogue).

La ligne de pilotage pneumatique 8, i.e. tuyau flexible ou analogue, servant au pilotage de la valve expiratoire 11 est connectée au circuit de gaz 3 interne, en aval (en 8.1) de la sortie de la turbine 2, ce qui permet une fermeture de la valve expiratoire 11, typiquement par action sur les moyens d'étanchéité flexibles de la valve expiratoire 11, pendant l'inspiration du patient, c'est-à-dire pendant les phases d'inspiration et/ou de plateau (i.e. pendant les durées Di et/ou Dp) selon le mode de réalisation considéré, grâce à la pression gazeuse provenant de la turbine 2 qui se propage via la ligne de pilotage pneumatique 8 jusqu'à la valve expiratoire 11.

Préférentiellement, les moyens d'étanchéité flexibles de la valve expiratoire 11 comprennent un ballonnet ou une membrane flexible/déformable ou analogue, et un orifice de mise à l'atmosphère ou évent en liaison avec l'atmosphère, pour contrôler le flux gazeux, c'est-à-dire pour bloquer toute sortie de gaz via ledit orifice de mise à l'atmosphère en empêchant ainsi toute sortie de gaz pendant les phases inspiratoires, ou à l'inverse libérer plus ou moins cet orifice pour autoriser alors le passage de gaz vers l'atmosphère, donc sa sortie du circuit patient 10 externe. Une telle architecture est classique.

Autrement dit, les moyens d'étanchéité flexibles sont agencés dans le corps de valve et coopèrent avec une partie de la paroi dudit corps de valve à la manière d'un clapet et d'un siège de clapet pour contrôler les flux gazeux au travers du corps de valve et leur libération à l'atmosphère.

Dans un mode de réalisation, la valve expiratoire 11, i.e. valve d'expiration, peut comprendre en outre une zone étanche comprise entre la membrane et le corps supérieur de la vanne 11 qui permet le pilotage en fonction de la pression qui est envoyée dans la zone étanche par le biais du piquage de pilotage.

Par ailleurs, la (les) mesure de pression proximale opérée(s) par le capteur de pression proximale 7 reflète(nt) la pression s'exerçant dans le circuit patient 10 au plus près du patient (en 7.2), c'est-à-dire au voisinage immédiat de ses voies respiratoires.

Quand la pression proximale mesurée au plus près du patient atteint la valeur de pression maximale (pré)réglée, c'est-à-dire le seuil de pression maximale ($P_{max}$) préfixé, voire quand un temps d'inspiration ($T_{max}$) maximum (pré)fixé ou réglé est atteint, les moyens de pilotage 4 contrôlent la turbine 2, plus précisément son moteur électrique, de sorte que celle-ci ralentisse et/ou freine, c'est-à-dire que les rotations du moteur 2 diminuent et/ou stoppent, mais tout en maintenant éventuellement une pression positive dans le circuit 10 à la valeur de pression de plateau $P_{pla}$ lorsqu'un tel plateau est souhaité

Comme déjà dit, les comparaisons entre les valeurs de pression proximale mesurée et de pression maximale préfixée, c'est-à-dire le seuil de pression maximale ($P_{max}$) préfixé, sont opérées par les moyens de pilotage 4, typiquement par un microprocesseur.

Par ailleurs, les moyens de pilotage 4 peuvent en outre comprendre un compteur temporel ou analogue pour déterminer les durées ou autres périodes de temps.

Du fait de la présence du dispositif anti-retour 6, toute diminution ou chute de pression n'est pas directement transmise au circuit patient 10 mais reste dans le circuit interne 3 et dans la ligne de pilotage 8 de la valve d'expiration 11, ce qui permet à la valve 11 de s'ouvrir, après la phase d'insufflation ou de plateau, puisque la pression s'exerçant dans le ballonnet ou sur la membrane de la valve 11 diminue, et dès lors de laisser le gaz s'échapper à l'atmosphère, donc le patient expirer au travers de ladite valve expiratoire 11 qui communique alors fluidiquement avec l'atmosphère, via son orifice de mise à l'atmosphère étant donné que le ballonnet ou la membrane n'assure plus l'étanchéité gazeuse.

L'expiration du patient se déroule alors de façon passive à travers la valve d'expiration 11 du circuit patient 10 et vers l'atmosphère, i.e. au travers du corps de valve et de l'orifice de mise à l'atmosphère.

Plus généralement, dans l'appareil de l'invention, l'utilisation de la pression proximale pour la synchronisation des phases inspiratoires et expiratoires du patient permet une meilleure sélectivité du système qui n'est plus influencée par le débit venant de la turbine 2 et par les résistances pneumatiques du circuit patient 10.

La détection de l'effort respiratoire du patient par l'appareil 1 se fait de manière plus précise et plus sensible, ce qui permet d'améliorer le traitement IPPB, notamment du fait d'une meilleure synchronisation de la délivrance de gaz à l'effort respiratoire du patient.

Dans le cas de l'utilisation d'une régulation PID, le passage d'une régulation de débit à une régulation de pression nécessite une réinitialisation de certains paramètres, tels que le facteur intégral, pour éviter la chute de la vitesse turbine pendant la phase de chargement du facteur intégral.

D'une façon générale, l'appareil de thérapie respiratoire selon l'invention est bien adapté à la fourniture d'une thérapie respiratoire de type IPPB aux patients en ayant besoin, typiquement aux personnes souffrant de problèmes ou pathologies respiratoires nécessitant d'améliorer leur drainage bronchique ; leur fonction respiratoire ; et leur recrutement pulmonaire, en particulier des zones pulmonaires mal ou non ventilées.

## Revendications

1. Appareil de thérapie respiratoire (1) pour fournir une assistance respiratoire intermittente par pression positive (IPPB) à une personne, comprenant :

   - une coque ou carcasse périphérique (1.1),
   - une turbine (2) motorisée pour fournir un gaz respiratoire sous pression,
   - un circuit de gaz (3) interne pour convoyer le gaz respiratoire délivré par la turbine (2),
   - un capteur de débit (5) agencé sur le circuit de gaz (3) interne,
   - un dispositif anti-retour (6) agencé dans le circuit de gaz (3) interne, en aval du capteur de débit (5),
   - un circuit patient (10) externe venant se raccorder fluidiquement au circuit de gaz (3) interne pour convoyer le gaz respiratoire provenant du circuit de gaz (3) interne,
   - un capteur de pression proximale (7) agencé pour permettre une mesure de la pression au sein du circuit patient (10) externe, et
   - des moyens de pilotage (4) raccordés au capteur de pression proximale (7) et à la turbine (2) motorisée, et configurés pour commander la turbine (2) en fonction d'au moins une mesure de pression opérée par le capteur de pression proximale (7) et d'au moins une mesure de débit opérée par le capteur de débit (5),

   et dans lequel la turbine (2), les moyens de pilotage (4), le circuit interne (3), le capteur de débit (5) et le dispositif anti-retour (6) sont agencés dans la coque ou carcasse périphérique (1.1),
   **caractérisé en ce que** les moyens de pilotage (4) sont configurés pour, lors de chaque phase inspiratoire de la personne, commander la turbine pour fournir un débit gazeux régulé décroissant, pendant une durée d'insufflation de gaz (Di) non-nulle suffisante pour atteindre un seuil de pression maximale ($P_{max}$) fixé.

2. Appareil selon la revendication 1, **caractérisé en ce qu'**il comprend en outre une valve expiratoire (11) agencée sur le circuit patient (10) externe, et une ligne de pilotage pneumatique (8) de la valve expiratoire (11) venant se raccorder fluidiquement (8.1) au circuit de gaz (3) interne entre la turbine (2) et le capteur de débit (5), et coopérant avec la valve expiratoire (11) pour contrôler l'ouverture ou la fermeture de ladite valve expiratoire (11).

3. Appareil selon la revendication 2, **caractérisé en ce que** le capteur de pression proximale (7) est agencé pour permettre une mesure de la pression au sein du circuit patient (10) externe via une ligne de mesure de pression (7.1) venant se raccorder au circuit patient (10) en aval (7.2) de la valve expiratoire (11).

4. Appareil selon la revendication 1, **caractérisé en ce que** lorsqu'une pression mesurée ($P_{mes}$) par le capteur de pression proximale est supérieure ou égale au seuil de pression maximale ($P_{max}$) préfixé, les moyens de pilotage (4) sont configurés pour commander la turbine pour fournir une pression de plateau ($P_{plat}$) correspondant au seuil de pression maximale ($P_{max}$) de manière à maintenir au moins une partie du circuit patient externe à ladite pression de plateau ($P_{plat}$), pendant une durée de plateau (Dp) non-nulle donnée.

5. Appareil selon la revendication 1, **caractérisé en ce que** le débit gazeux régulé décroit proportionnellement à l'augmentation de la pression jusqu'à atteindre le seuil de pression maximale ($P_{max}$).

6. Appareil selon la revendication 1, **caractérisé en ce que** les moyens de pilotage (4) commandent un ralentissement ou un freinage de la turbine (2) lorsque lesdits moyens de pilotage (4) déterminent que la pression proximale mesurée atteint le seuil de pression maximale ($P_{max}$).

**7.** Appareil selon la revendication 1, **caractérisé en ce que** les moyens de pilotage (4) sont configurés pour, lors de chaque phase inspiratoire de la personne, commander la turbine pour fournir un débit gazeux régulé décroissant entre un débit initial ($Q_{init}$) et un débit final ($Q_{fin}$), avec $Q_{init} > Q_{fin}$.

**8.** Appareil selon la revendication 7, **caractérisé en ce que**, le débit final ($Q_{fin}$) est égal à une proportion (x%) du débit initial ($Q_{init}$) comprise entre 0 et 70% du débit initial ($Q_{init}$).

**9.** Appareil selon la revendication 8, **caractérisé en ce que** le débit objectif ($Q_{objectif}$) est égal à une proportion du débit initial ($Q_{init}$) et par ailleurs à la montée en pression du circuit patient (10) selon la formule suivante :

$$Q_{objectif} = (100\% - x\%) \cdot Q_{init} \cdot (P_{max} - P_{mes}) / P_{max} + x\% \cdot Q_{int}$$

où :

- $Q_{objectif}$ correspond à la valeur de débit régulée au cours de l'inspiration considérée.
- $Q_{init}$ correspond à la valeur de débit initial, c'est-à-dire un débit réglé ($Q_{réglé}$), de préférence le débit initial ($Q_{init}$) est égal à : $y \cdot (Q_{réglé})$ avec y compris entre 0,5 et 2, de préférence entre 1 et 2.
- x% correspond à la proportion du débit initial ($Q_{init}$) comprise entre 0 et 70%, de préférence entre 30 et 70%, par exemple de l'ordre de 50%,
- $P_{max}$ est la pression maximale fixée.
- $P_{mes}$ est la pression proximale mesurée par le capteur de pression proximale.

**10.** Appareil selon l'une des revendications 1 ou 7 à 9, **caractérisé en ce que** le seuil de pression maximale ($P_{max}$) est compris entre 10 et 50 cmH2O et/ou le débit initial ($Q_{init}$) est compris entre 1 et 200 L/min.

**11.** Appareil selon la revendication 8, **caractérisé en ce que** la proportion (x%) est comprise entre 30 et 70% du débit initial ($Q_{init}$).

**12.** Appareil selon la revendication 10, **caractérisé en ce que** le débit initial ($Q_{init}$) est compris entre 3 et 150 L/min.

**13.** Appareil selon la revendication 1, **caractérisé en ce que** les moyens de pilotage (4) comprennent au moins un microprocesseur.

**14.** Appareil selon la revendication 1, **caractérisé en ce que** la turbine (2) motorisée comprend un moteur électrique piloté par les moyens de pilotage (4).

**15.** Appareil selon la revendication 1, **caractérisé en ce que** le capteur de pression proximale (7) est configuré pour permettre une mesure de la pression au sein du circuit patient externe (10) entre la valve expiratoire (11) et une interface respiratoire (20) assurant les échanges gazeux avec le patient.

Fig. 1

Fig. 2

Fig. 3

ART ANTERIEUR

INVENTION

Fig. 4

Europäisches Patentamt

European Patent Office

Office européen des brevets

**RAPPORT DE RECHERCHE EUROPEENNE**

Numéro de la demande

EP 26 15 0403

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | DE 10 2014 001218 A1 (WEINMANN EMERGENCY MEDICAL TECHNOLOGY GMBH & CO KG [DE]) 30 juillet 2015 (2015-07-30) * le document en entier, et en particulier les paragraphes [0032], [0036], [0046]-[0047], [0049], les revendications 15, 21 et 22, et les figures 1-2 * ----- | 1-15 | INV. A61M16/00 A61M16/20 |
| A,D | WO 2015/110098 A1 (WEINMANN EMERGENCY MEDICAL TECHNOLOGY GMBH & CO KG [DE]) 30 juillet 2015 (2015-07-30) * le document en entier et en particulier les figures 1 à 4 * ----- | 1-15 | |
| A,D | EP 1 502 619 A1 (AIROX [FR]) 2 février 2005 (2005-02-02) * le document en entier et en particulier la figure 1 * ----- | 1-15 | |
| A,D | US 2013/047983 A1 (ANDRIEUX CLAUDE [FR] ET AL) 28 février 2013 (2013-02-28) * le document en entier et en particulier les figures 5 et 6 * ----- | 1-15 | **DOMAINES TECHNIQUES RECHERCHES (IPC)** A61M |
| A,D | US 2024/165351 A1 (CIPOLLONE JOSEPH [US] ET AL) 23 mai 2024 (2024-05-23) * le document en entier et en particulier les figures * ----- | 1-15 | |
| A,D | US 2008/000478 A1 (MATTHIESSEN HANS [DE] ET AL) 3 janvier 2008 (2008-01-03) * le document en entier et en particulier les figures * ----- | 1-15 | |

Le présent rapport a été établi pour toutes les revendications

| Lieu de la recherche | Date d'achèvement de la recherche | Examinateur |
|---|---|---|
| Munich | 10 février 2026 | Azaïzia, Mourad |

**ANNEXE AU RAPPORT DE RECHERCHE EUROPEENNE
RELATIF A LA DEMANDE DE BREVET EUROPEEN NO.**  EP 26 15 0403

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-02-2026

| Document brevet cité au rapport de recherche | Date de publication | Membre(s) de la famille de brevet(s) | Date de publication |
|---|---|---|---|
| DE 102014001218 A1 | 30-07-2015 | AUCUN | |
| WO 2015110098 A1 | 30-07-2015 | CN 105934260 A | 07-09-2016 |
| | | DE 102014000884 A1 | 23-07-2015 |
| | | EP 3096827 A1 | 30-11-2016 |
| | | RU 2016134208 A | 05-03-2018 |
| | | WO 2015110098 A1 | 30-07-2015 |
| EP 1502619 A1 | 02-02-2005 | AT E390161 T1 | 15-04-2008 |
| | | DE 602004012672 T2 | 16-04-2009 |
| | | EP 1502619 A1 | 02-02-2005 |
| | | ES 2301937 T3 | 01-07-2008 |
| | | FR 2858236 A1 | 04-02-2005 |
| | | US 2005039748 A1 | 24-02-2005 |
| | | US 2008011300 A1 | 17-01-2008 |
| | | US 2010186744 A1 | 29-07-2010 |
| US 2013047983 A1 | 28-02-2013 | CA 2738224 A1 | 08-04-2010 |
| | | EP 2331212 A2 | 15-06-2011 |
| | | US 2010078016 A1 | 01-04-2010 |
| | | US 2013047983 A1 | 28-02-2013 |
| | | WO 2010039371 A2 | 08-04-2010 |
| US 2024165351 A1 | 23-05-2024 | CA 3216818 A1 | 20-10-2022 |
| | | CN 117440847 A | 23-01-2024 |
| | | EP 4323041 A1 | 21-02-2024 |
| | | JP 2024518030 A | 24-04-2024 |
| | | US 2024165351 A1 | 23-05-2024 |
| | | WO 2022221863 A1 | 20-10-2022 |
| US 2008000478 A1 | 03-01-2008 | DE 102006030520 B3 | 21-06-2007 |
| | | US 2008000478 A1 | 03-01-2008 |

EPO FORM P0460

Pour tout renseignement concernant cette annexe : voir Journal Officiel de l'Office européen des brevets, No.12/82

**EP 4 782 038 A1**

## RÉFÉRENCES CITÉES DANS LA DESCRIPTION

**Documents brevets cités dans la description**

- EP 4079356 A
- DE 102014001218
- WO 2015110098 A
- EP 1502619 A
- US 2013047983 A
- US 2024165351 A
- US 2008000478 A
- EP 2947328 A